# EUROPEAN PATENT APPLICATION

(11) **EP 0 739 638 A1**
(43) Date of publication of application: **30.10.1996**
(21) Application number: 95928621.2
(22) Date of filing: 21.08.1995
(51) Int. Cl.: A61M 5/31, A61L 2/06

(54) **METHOD OF STERILIZING PREFILLED SYRINGE MEDICINES**

(30) Priority: 19.08.1994 JP 218103/94
(71) Applicant: EIKEN CHEMICAL CO., LTD., 2 Bunkyo-ku, Tokyo 113 (JP)
(72) Inventor: MATSUMOTO, Yasuyuki, Shimotsuga-gun Tochigi-ken 329-01 (JP); SAITO, Masahiro, Shimotsuga-gun Tochigi-ken 329-01 (JP); MORIKAWA, Junji, Kita-ku Tokyo 114 (JP); TAKANO, Hiroshi, Kita-ku Tokyo 114 (JP)
(74) Representative: Crouch, David John
(86) International application number: JP9501644
(87) International publication number: WO9605877

(57) **Abstract**

The invention provides a method of sterilizing a chemical and an injector in a prefilled syringe and keeping them in sterilized condition, said method comprising mounting a liquid leakage preventive cap (1) on a tip end of an injector (2) in a prefilled syringe, filled with a chemical and mounting a sliding plug (3) in an opening at a rear end of the injector to prevent liquid leakage of the prefilled syringe, securing the prefilled syringe, which mounts thereto the liquid leakage preventive cap (1) and the sliding plug (3), by means of a securing container (6), sealing the securing container (6), to which the prefilled syringe (2) is fixed, by means of a poromeric and bacteria impermeable nonwoven paper or fabric to sterilize a chemical (5) in the prefilled syringe (2), the prefilled syringe (2) and the securing container at the same time. The securing container (6) is enclosed by a bag-shaped nonwoven paper or fabric (7). Alternatively, the securing container (6) may have an opening, and the opening may be sealed by the nonwoven paper or fabric (11). According to the invention, it is possible to manufacture a prefilled syringe kit readily at low cost in a sterilized manner and keep the sterilized condition.

## Description

### Technical Field

The present invention relates to a method for aseptically producing medical supplies of an injector filled with liquid medicines (hereinafter "prefilled syringe kit"). Particularly, it relates to a method for sterilizing not only an inner liquid medicine, hut also an injector itself filled with the liquid medicine in advance (hereinafter "prefilled syringe") and the inside of outer packagings simultaneously in an single operation, as well as for maintaining its asepsis. More particularly, it relates to a method for asepticaliy producing prefilled syringe kits easily and cheaply.

Conventionally, there have been provided liquid medicines (injections) which are sterilized and filled in a vial or an ampoule. But in recent years, prefilled syringe kits have been remarkably used, because of their easy handling. It has also accelerated the the spread of prefilled syringes that the material of syringes has been changed from conventional glass to synthetic resins (plastics) having a high impact-resistance.

Generally, in aseptically producing prefilled syringe kits, the production process comprises the steps of liquid medicine preparation, filtration, charging of medicine into a syringe, capping, sterilization, inspection, and packaging. The liquid medicine must be aseptic. Furthermore according to the "1993 Supplement to DRUG APPROVAL AND LICENSING PROCEDURES IN JAPAN" (published on 30th September, 1993 by Yakugyo Jiho Co., Ltd. and edited by Nippon Kouteisho Kyokai), it is prescribed that "With respect to guaranteed asepsis, not only the content medicine, but also the needle and the injection cylinder of which package was aseptically opened and which were aseptically removed out from the package must conform to the sterility test." Thus, not only the the content medicine, but also the outer surface of the injector and the inside of outer package must be now aseptic. Therefore, in order to keep the whole kit aseptic, it is now required to carry out the production steps aseptically in a sterile room (clean room).

However, in order to carry out these steps aseptically, it is necessary that the operation room is a sterile room for avoiding a microbial contamination from the operation room, and moreover, the operations are required to be fully automated for preventing a microbial contamination during the operations. For the above mentioned purposes, arrangements such as facilities and equipment are very expensive. In addition, a long period of time required to install and complete these arrangements including a preparation period, complicated operations, and running cost as well as expenses and labor in maintenance should be considered.

There are a lot of kinds of sterilization method such as heat methods, irradiation methods, gaseous methods, and a medicament methods. There are a lot of kinds of the heat methods such as a boiling water sterilization, a flow steam sterilization, a steam under pressure (autoclave) sterilization, and a dry heat sterilization. Normally, in the sterilization of medicines, a autoclave sterilization is used, wherein the sterilization is carried out in saturated steam at adequate temperature of 115 °C to 126 °C for 30 min to 15 min. In this autoclave sterilization, since a filled liquid medicine and a gas remaining in the syringe (air or substituted nitrogen) are expanded due to a high temperature of autoclave, it tends to remove a cap from the tip of the syringe and it tends to remove a slide plug from the rear end of the syringe to leak the liquid medicine. Conventionally, in order to avoid these leakage, it was proposed to increase or decrease pressure corresponding to the change of the inner pressure of the syringe during the autoclave sterilization, or to fix the cap on the tip of the syringe and the slide plug on the rear end thereof to fastening device so as not to loosen (Japanese Patent Publication No. Hei 6-34827, Japanese Patent Laid-open No. Hei 5-253296, Japanese Patent Laid-open No. Hei 3-141951, and Japanese Utility Model Publication No. Hei 5- 74544).

But, in the former method, since the autoclave equipment necessitates more pressure-resistant construction, the facilities become large-sized, require a great deal of installation space, and are more expensive than the usual autoclave equipment (Japanese Patent Laid-open No. Hei 3-141951). In the latter method, complicated preparatory operations are necessary, such as requiring a special device for fastening the tip rubber cap and the rear end rubber plug, and fixing the syringe before the sterilization (Japanese Utility Model Publication No. Hei 6-124, and Japanese Patent Laid-open No. Hei 5-253296).

The above described conventional methods relate to a sterilization of the liquid medicine in a prefilled syringe and the whole prefilled syringe. But, in order to guarantee the asepsis of the whole prefilled syringe kit within the package, it is further necessary to handle and package the prefilled syringe aseptically after sterilization. That is, the sterilized prefilled syringe must be aseptically taken out from a sterilizer so as not to be contaminated by microorganisms, and it must be packaged by sterilized packagings in a sterile room. Further, plastic syringes have possibilities of troubles such as being softened and deformed under high temperature and high pressure. Thus, in order to avoid these disadvantages, it is required to discuss not only a sterilizer, a heating method, a packaging style, and a packaging method, but also a method of preventing the deformation of plastic containers. Therefore, in order to produce prefilled syringe kits aseptically, it was necessary to establish an aseptic production line including a sterile room and aseptic manipulation, and sufficient time and expenses were required.

Accordingly, it is an object of the present invention to provide a method for aseptically producing prefilled syringe kits, in which an autoclave sterilization of prefilled syringes is carried out without requiring a large amount of expenses and special facilities as well as without requiring long preparation period and complicated operations. It is an another object of the present invention to provide prefilled syringe kits that are marketable hygienically in this thus obtained form.

### Summary of the Invention

The present invention relates to a method for sterilizing liquid medicine and an injector of a prefilled syringe in one sterilization process, as well as for maintaining its asepsis.

According to the present invention, there is provided a method for sterilizing a liquid medicine in a prefilled syringe and an injector of the prefilled syringe, as well as for maintaining its asepsis, comprising the steps of: mounting a liquid leakage prevention cap on a tip of an injection cylinder of the prefilled syringe and a slide plug at an opening of a rear end thereof so as to prevent liquid leakage from the prefilled syringe; setting the prefilled syringe including the liquid leakage prevention cap and the slide plug to a setting container; wrapping or sealing the setting container set with the prefilled syringe by means of air-permeable and bacteria-impermeable unwoven paper or unwoven cloth (hereinafter referred as "unwoven paper"); and sterilizing the liquid medicine in the prefilled syringe, the prefilled syringe, and the setting container simultaneously.

Said setting container can be wrapped in baggy unwoven paper. Alternatively, said setting container has an opening, and said opening may be sealed with unwoven paper. Said sterilization is carried out in an autoclave. Said prefilled syringe and said prefilled syringe setting container are made of a heat-resistant and pressure-resistant material which can withstand autoclave sterilization.

### Brief Description of the Drawings

FIG. 1 is a side view and a top plan view of the prefilled syringe used in the present invention.

FIG. 2 is a perspective view illustrating the prefilled syringe setting container and the sterilizing bag.

FIG. 3 is a side view and a top plan view of the prefilled syringe kit including the the prefilled syringe and the prefilled syringe setting container used in the present invention.

### Best Mode of Carrying out the Invention

A sterilizing container used in the method of the present invention is described with reference to the accompanying drawing, but the present invention is not limited to the illustrated embodiments.

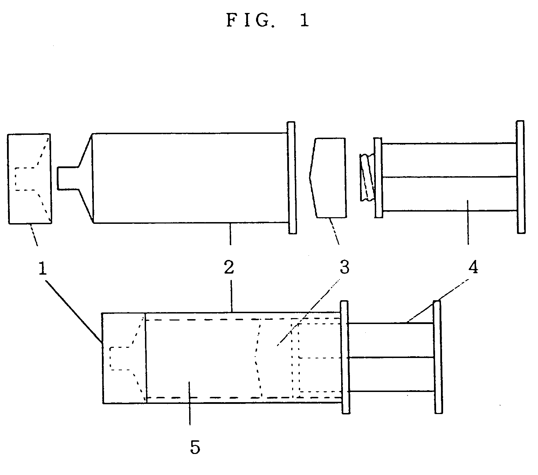

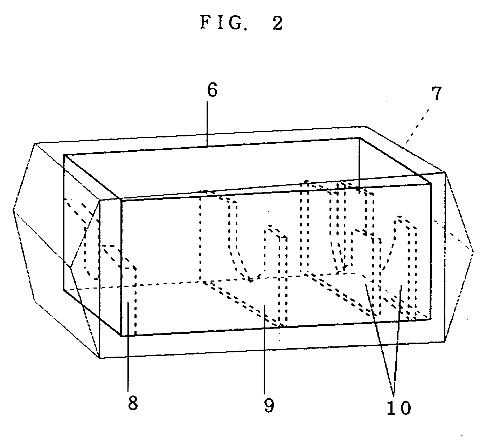

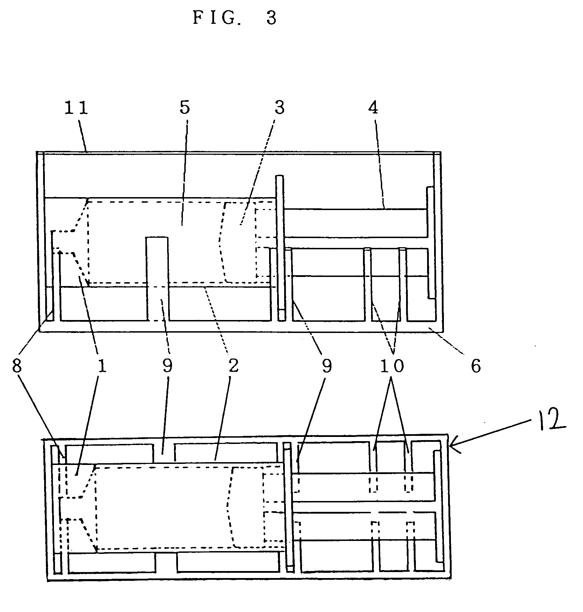

In the present invention, a prefilled syringe shown in FIG. 1 and a sterilizing container 12 shown in FIG. 2 or FIG. 3 are used.

An injector used in the present invention comprises an injection cylinder 2, a piston 4, a liquid leakage prevention cap 1, and a slide plug 3. A prefilled syringe comprises said injector and a liquid medicine 5 included in the injector. In order to prevent liquid leakage from the prefilled syringe, the liquid leakage prevention cap 1 is mounted on a tip (nozzle) of the injection cylinder 2, and the slide plug 3 is mounted at an opening of the rear end of the injection cylinder 2. Material of the liquid leakage prevention cap 1 and the slide cap 3 may be a generally used elastic substance. In the present invention, rubber, especially well slidable rubber coated with silicon or teflon, is preferably used. The cap 1 may be fitted or screwed on the nozzle of the injector 2. The slide plug 3 is directly connected with the piston 4 and is moved corresponding to the motions of the piston 4. The slide plug 3 is preferably fitted to the piston 4, because the piston 4 is moved in the axial direction of the syringe in using the prefilled syringe. It is also possible to seal the opening of the rear end of the injection cylinder 2 with the slide plug 3 without the piston 4, thereby forming an ampoule-like construction.

The sterilizing container 12 comprises a prefilled syringe setting container 6 and unwoven paper.

The prefilled syringe setting container 6 is a wrapable box as shown in FIG. 2 or a sealable box having an opening shown in FIG. 3. The prefilled syringe setting container 6 is provided with a liquid leakage prevention cap receiver 8 for receiving the liquid leakage prevention cap 1, an injection cylinder receiver 9 for receiving the injection cylinder 2, and a piston receiver 10 for receiving the piston 4 of the prefilled syringe. The liquid leakage prevention cap receiver 8, the injection cylinder receiver 9, and the piston receiver 10 may be of any type as long as they can set the prefilled syringe so that it may not be moved during sterilization operation and transportation.

Said prefilled syringe setting device 6 is prevented from the microbial invasion by means of unwoven paper. Such unwoven paper is air-permeable and bacteria-impermeable unwoven paper (unwoven paper or unwoven cloth), and this air-permeable and bacteria-impermeable unwoven paper means unwoven paper which can permeate gases such as air and steam, but which does not permeate microorganisms, colloidal particles, and other particles of micrometer-size.

A bag is formed by such unwoven paper 7, and wraps the whole prefilled syringe setting container 6 as shown in FIG. 2. The prefilled syringe setting container 6 may be partially (only the portion where sterilization is required) wrapped in unwoven paper 7. As shown in FIG. 3, the opening of the prefilled syringe setting container 6 set with the prefilled syringe may be sealed by means of unwoven paper 11. In this case, the unwoven paper 11 is adhered or bonded to the opening of the prefilled syringe setting container 6. Since the prefilled syringe setting container 6 set with the prefilled syringe is covered with this unwoven paper in whole or partially, the asepsis of the inside of sterilizing container after sterilization is maintained.

The prefilled syringe and the prefilled syringe setting container 6 may be made of various types of material having a heat-resistance and pressure-resistance which can withstand autoclave sterilization. For example, if the material is a plastic, it may be made of cross-linked polymers etc., such as polysulfone, polyether-sulfone, polyamide, polycarbonate, polymetylpentene, polyesther, polypropylene, and polyetylene. Particularly, transparent polycarbonate or polypropylene is preferably used in the present invention, because the inside of the container is visible. By using a transparent material, the visual inspection during production can be easily carried out even after sterilization.

The thus obtained sterilizing container 12 is sterilized in an autoclave. Since the prefilled syringe setting container 6 set with the prefilled syringe is covered with said unwoven paper in whole or partially, the asepsis after sterilization is maintained. The autoclave sterilization is carried out under the normal conditions (at 115 °C to 126 °C, in saturated steam, for 30 min. to 15 min.).

The present invention can be applied to various medicines when such medicines are heat-resistant such that they are not heat-denaturated by an autoclave. The present invention is convenient for a contrast medium having very high viscosity, because a sucking and dispensing operation of the contrast medium from a vial or an ampoule into an injector is complicated, and the operation takes much time.

The aseptic prefilled syringe kit is produced as follows. The setting container 6 is formed of a heat-resistant and pressure-resistant plastic; material which can withstand high temperature and high pressure during autoclave sterilization. The prefilled syringe assembled with the liquid medicine 5, the liquid leakage prevention cap 1 and the slide plug 3 is set in this setting container 6 in order to prevent the removal of the liquid leakage prevention cap 1 and the slide plug 3 from the prefilled syringe during autoclave sterilization. The whole setting container 6 is wrapped in the sterilizing bag 7, or the setting container is partially sealed with the unwoven paper 11 which is impermeable to microorganisms, colloidal particles, and other particles of micrometer size, but is permeable to steam. Then, autoclave sterilization is carried out to obtain the aseptic; prefilled syringe kits.

The present invention will be described below in detail with reference to examples, but the present invention is not limited to the examples.

### Example 1

A heat-resistant bacteria (Bacillus stearothermophilus ATCC 12980) generally used as a biological indicator for confirming the sterile effect of the autoclave was added to a liquid medicine previously prepared and filtrated by means of a membrane filter (0.22 micrometer: made by MILLIPORE Inc.) to obtain a liquid medicine containing said bacteria of about 10⁵/ml. The injector of FIG. 1 was filled with said liquid medicine and was capped and plugged. Said prefilled syringe was loaded in a setting container 6 made of transparent polyethylene cross-linked polymer (Brand Name: CZ; made by DAIKYO SEIKO Inc.). The setting container 6 was wrapped in a sterilizing bag 7 (see FIG. 2). The bag 7 was sealed. Then an autoclave sterilization was carried out at 121 °C for 20 min.

The thus produced liquid medicine in the prefilled syringe kit underwent the sterility test (The Pharmacopeia of Japan 12th Edition, General Tests Processes and Apparatus, Sterility Test), and it was confirmed that the liquid medicine was aseptic and that the sterilization was complete.

### Example 2

About 10⁵ of Bacillus stearothermophilus ATCC 12980 was applied on the inside of a sterilizing bag 7. The bag 7 was sealed. The autoclave sterilization was carried out at 121 °C for 20 min.

After cooling, aseptic physiological saline solution was aseptically poured into the sterilizing bag 7. The inside of the bag was washed by the solution to obtain a sample solution. The thus obtained sample solution underwent the sterility test. It was confirmed that the sample solution was aseptic and that the sterilization was complete.

### Example 3

About 10⁵ of Bacillus stearothermophilus ATCC 12980 was applied on the inside of a setting container 6. Then, the container was sealed by means of air-permeable and bacteria-impermeable unwoven paper 11 (FIG. 3). Autoclave sterilization was carried out at 121 °C for 20 min.

After cooling, aseptic physiological saline solution was aseptically poured into the sterilized container, and the inside of the container was washed to obtain the sample solution. The thus obtained sample solution underwent the sterility test. It was confirmed that the sample solution was aseptic and that the sterilization was complete.

### Example 4

As a biological indicator for confirming the sterile effect of the autoclave, a heat-resistant bacteria (Bacillus stearothermophilus ATCC 12980) was added to a liquid medicine previously prepared and filtered by means of the membrane filter (0.22 micrometer) to obtain a liquid medicine containing said bacteria of about 10⁵/ml. The injector of FIG. 1 was filled with said liquid medicine and was capped and plugged. Said prefilled syringe was loaded in a setting container 6 made of transparent polycarbonate. The setting container 6 was wrapped in a sterilizing bag 7 on whose inside about 10⁵ of Bacillus stearothermophilus ATCC 12980 was applied. The bag was sealed. Then, an autoclave sterilization was carried out at 121 °C for 20 min.

The thus produced prefilled syringe kit underwent the sterility test. It was confirmed that the liquid medicine and the inside of the sterilizing bag were aseptic.

### Example 5

As a biological indicator for confirming the sterile effect of the autoclave, a heat-resistant bacteria (Bacillus stearothermophilus ATCC 12980) was added to a liquid medicine previously prepared and filtered by means of the membrane filter (0.22 micrometer) to obtain a liquid medicine containing said bacteria of about 10⁵/ml. The injector of FIG. 1 was filled with said liquid medicine and was capped and plugged. Said prefilled syringe was loaded in a setting container 6 (sterilizing container 12) which was made of transparent polyethylene cross-linked polymer (CZ), and the inside of the sterilizing container was applied with about 10⁵ of Bacillus stearothermophilus ATCC 12980. The container 6 was sealed by means of unwoven paper 11 to make sterilizing container 12 as shown in FIG. 3, and autoclave sterilization was carried out at 121 °C for 20 min.

The liquid medicine and the inside of the container of the produced prefilled syringe kit underwent the sterility test, and it was confirmed that the liquid medicine and the inside of the sterilizing container were aseptic.

According to the present invention, no large amount of expenses, time and labor for the special facilities are required, The provisions of the Pharmaceutical Affairs Law of Japan which guarantee the asepsis of the liquid medicine and the inside of the package of the prefilled syringe are easily accomplished.

The setting container of the present invention withstands autoclave sterilization, and prevents liquid medicine leakage from the prefilled syringe and removal of the liquid leakage prevention cap and slide plug. This container becomes a sterilizing container which maintains asepsis by being enclosed in a sterilizing bag or being covered with unwoven paper. Since this sterilizing container is not only permeable to air, but also impermeable to microorganisms, it can maintain the asepsis of the inside of the sterilizing container after sterilization. The sterilizing container can be used as a package (prefilled syringe kit), and is simply packed in an outer packaging box to provide a medical product on the market.

According to the present invention, although a setting container and a sterilizing container are necessary, it costs only a minimum. Moreover, since the sterilizing container itself is used also as a packaging container, there is little newly added expense. Further, since the the present invention can be applied to the conventional art, the conventional production line is available with a little modification, and therefore, no preparation period for modifying the production line is required, which makes it possible to carry out the present invention readily.

In other words, according to the present invention in which the prefilled syringe is set to the sterilizing container and is applied to an autoclave sterilization, there is provided a method for producing a prefilled syringe kit, which does not require conventional complicated operations as well as a great deal of expenses and time, resolves all of these problems, and guarantees the asepsis of the whole prefilled syringe kit easily and cheaply, and there is also provided a prefilled syringe kit in a compact packaging manner.

## Claims

1. A method for sterilizing a liquid medicine in a prefilled syringe and the prefilled syringe, as well as for maintaining their asepsis, comprising the steps of:
mounting a liquid leakage prevention cap (1) on a tip of an injection cylinder (2), and a slide plug (3) at an opening of a rear end of the injection cylinder so as to prevent liquid leakage from the prefilled syringe;
setting the prefilled syringe including the liquid leakage prevention cap (1) and the slide plug (3) to a setting container (6) so as to prevent removal of the said cap (1) and the said plug (3);
sealing the setting container (6) set with the prefilled syringe (2) by means of air-permeable and bacteria-impermeable unwoven paper or unwoven cloth; and
sterilizing the liquid medicine (5) in the prefilled syringe (2), the prefilled syringe, and the setting container.

2. The method as defined in claim 1, wherein said setting container (6) is wrapped in baggy unwoven paper or unwoven cloth (7).

3. The method as defined in claim 1, wherein said setting container (6) has an opening, and said opening is sealed with unwoven paper or unwoven cloth (11).

4. The method as defined in claim 1, wherein said sterilization is carried out by means of autoclave.

5. The method as defined in claim 1, wherein said prefilled syringe and said setting container are made of a heat-resistant and pressure-resistant material which can withstand autoclave sterilization.
